# EUROPEAN PATENT SPECIFICATION

(11) **EP 2 154 238 B1**
(45) Date of publication and mention of the grant of the patent: **25.01.2012**
(21) Application number: 08764463.9
(22) Date of filing: 21.05.2008
(51) Int. Cl.: C12N 1/20, C12R 1/225, A23C 11/10

(54) **NOVEL LACTIC ACID BACTERIUM STRAIN, FERMENTED FOOD/BEVERAGE, AND METHOD FOR PRODUCTION OF FERMENTED FOOD/BEVERAGE**
NEUARTIGER MILCHSÄUREBAKTERIENSTRANG, FERMENTIERTES NAHRUNGSMITTEL/GETRÄNK UND VERFAHREN ZUR HERSTELLUNG EINES FERMENTIERTEN NAHRUNGSMITTELS/GETRÄNKS
NOUVELLE SOUCHE DE BACTÉRIE LACTIQUE, ALIMENT/BOISSON FERMENTÉ, ET PROCÉDÉ DE PRODUCTION D'ALIMENT/DE BOISSON FERMENTÉ

(30) Priority: 31.05.2007 JP 2007145678
(43) Date of publication of application: 17.02.2010
(73) Proprietor: Kagome Co., Ltd, Nagoya-shi, Aichi 460-0003 (JP)
(72) Inventor: SUZUKI, Shigenori, Nasushiobara-shi Tochigi 329-2762 (JP); TAKEDA, Masahiko, Nasushiobara-shi Tochigi 329-2762 (JP); INOUE, Takuro, Nasushiobara-shi Tochigi 329-2762 (JP); KAWASAKI, Rika, Nasushiobara-shi Tochigi 329-2762 (JP); ARAKAWA, Chinatsu, Nasushiobara-shi Tochigi 329-2762 (JP); KATO, Ikuo, Nasushiobara-shi Tochigi 329-2762 (JP); YAKABE, Takafumi, Nasushiobara-shi Tochigi 329-2762 (JP)
(74) Representative: Desaix, Anne
(86) International application number: PCT/JP2008/059340
(87) International publication number: WO 2008/146676

(56) References cited:
- WO-A1-2006/093267
- WO-A2-03/033681
- JP-A- 2004 290 012
- ANGELES A G ET AL: "Growth and activity of lactic acid bacteria in soymilk. I. Growth and acid production" J.MILK FOOD. TECHNOL 1971, vol. 34, no. 1, 1971, pages 30-36, XP002040396
- KANEKO S. ET AL.: 'Nyusankin o Riyo shita Hakko Tofu no Kaihatsu ni Kansuru Kenkyu (1st report)' RESEARCH REPORT OF FOOD TECHNOLOGY RESEARCH INSTITUTE OF NAGANO PREFECTURE no. 24, 01 October 1996, pages 63 - 66
- MATSUOKA H.: 'Daizu Cheese no Seizoho Oyobi Jukusei Kiko' JOURNAL OF THE JAPANESE SOCIETY FOR FOOD SCIENCE AND TECHNOLOGY vol. 42, no. 11, 15 November 1995, pages 945 - 951, XP008122096

### SEQUENCE LISTING

<110> KAGOME CO., LTD
<120> NEW LACTIC ACID BACTERIA STRAIN, FERMENTED FOOD OR DRINK, AND METHOD FOR PRODUCING THE FERMENTED FOOD OR DRINK
<130> B08572 -AD/SL/CAL
<140> EP 08764463.9
   <141> 2008-05-21
<150> JP 2007-145678
   <151> 2007-05-31
<160> 1
<170> PatentIn version 3.3
<210> 1
   <211> 495
   <212> DNA
   <213> Lactobacillus pentosus FERM BP-10958
<220>
   <221> misc_feature
   <222> (22)..(22)
   <223> n is a, c, g, or t
<400> 1
   cgtggctttc tggttaaata cngtcaatac ctgaacagtt actctcagat atgttcttct     60
   ttaacaacag agttttacga gccgaaaccc ttcttcactc acgcggcgtt gctccatcag    120
   actttcgtcc attgtggaag attccctact gctgcctccc gtaggagttt gggccgtgtc    180
   tcagtcccaa tgtggccgat taccctctca ggtcggctac gtatcattgc catggtgagc    240
   cgttacccca ccatctagct aatacgccgc gggaccatcc aaaagtgata gccgaagcca    300
   tctttcaaac tcggaccatg cggtccaagt tgttatgcgg tattagcatc tgtttccagg    360
   tgttatcccc cgcttctggg caggtttccc acgtgttact caccagttcg ccactcactc    420
   aaatgtaaat catgatgcaa gcaccaatca ataccagagt tcgttcgact tgcatgtatt    480
   aggcacgccg ccagc                                                     495

## Description

### BACKGROUND OF THE INVENTION

### Field of the Invention

The present invention relates to a new lactic acid bacteria strain, fermented food or drink products, and methods for producing the fermented food or drink.

Priority is claimed on Japanese Patent Application No. 2007-145678, filed on May 31, 2007.

### Description of the Related Art

Lactic acid bacteria have been used for producing various fermented food and drink products, and some of the bacteria per se have excellent physiological activities, such as an intestinal regulatory activity and inhibitory activity against pathogenic bacteria. Such useful lactic acid bacteria is utilized for the production of the food or drink product, and furthermore excellent fermented food or drink products designed to be good for a person's health can be produced by maintaining living lactic acid bacteria in the fermented food or drink products.

On the other hand, foods and drinks which processed vegetative raw materials such as vegetables have been developed vigorously, because insufficient vegetable intake within Western-style dietary habits has been a big problem.

In such a situation, fermented food or drink products, which are produced by fermentation of vegetative raw materials with lactic acid bacteria, have been gaining attention. These fermented food or drink products are given a distinctively excellent taste, and these products can not only cover such insufficient vegetable intake but also provide useful lactic acid bacteria in a viable condition.

Methods for producing fermented food or drink products, which are produced by fermentation of vegetative raw materials with lactic acid bacteria, have been disclosed. For example, a method of lactic acid fermentation by adding dairy products to vegetative raw materials (see Patent Documents 1 and 2), a method of lactic acid fermentation by adding sugar (in particular, lactose) to vegetative raw materials (see Patent Documents 3 and 4), and a method of using lactic acid bacteria capable of fermenting vegetative raw materials are disclosed (see Patent Documents 5, 6, and 7).
Patent Document 1: Japanese Unexamined Patent Application, First Publication No. Sho 60-248131
Patent Document 2: Japanese Unexamined Patent Application, First Publication No. Hei 1-179646
Patent Document 3: Japanese Unexamined Patent Application, First Publication No. Hei 1-247035
Patent Document 4: Japanese Unexamined Patent Application, First Publication No. Hei 7-236417
Patent Document 5: Japanese Unexamined Patent Application, First Publication No. Hei 5-84065
Patent Document 6: Japanese Unexamined Patent Application, First Publication No. Hei 5-84066
Patent Document 7: Japanese Laid-Open Patent Application No. 2007-37503

However, existing methods have a problem which limits fermentation conditions. Since lactose contents are extremely low, soybeans are difficult to fully ferment without the use of lactose. Therefore it is difficult to adjust the taste of fermented food and drink products, if soybeans are used as main raw materials. As curd forming is also difficult without sufficient fermentation, for example, it is difficult to produce solid fermenting products having an adjusted flavor, and there is a problem in that the obtained fermenting products have limited variety.

Moreover, there are problems in that the flavor of fermented food and drink products using lactic acid bacteria which can ferment with vegetative raw materials is not sufficiently improved, and furthermore the stability of quality during refrigeration storage is insufficient.

By contrast, lactic acid bacteria, which belong to *Lactobacillus pentosus* such as *Lactobacillus pentosus* strain JCM1558^{T}, can ferment soybeans without the use of lactose. However, there are no strains which have both curd forming ability and quality stability during refrigeration storage. Moreover there is a problem that the fermented product has pickle-like odors.

### SUMMARY OF THE INVENTION

The present invention has been achieved in consideration of the above situation with an object of providing new lactic acid bacteria having excellent flavor, less quality variation after refrigeration storage, and a high curd forming ability during fermentation, providing fermented food or drink produced by fermentation using the new lactic acid bacteria, and providing methods for producing fermented food or drink products using the new lactic acid bacteria.

The present invention includes the following aspects.
*(1) Lactobacillus pentosus* strain FERM BP-10958.
(2) A method for producing a fermented food or drink product including the step of fermenting using the *Lactobacillus pentosus* according to (1).
(3) A fermented food or drink product obtained by fermentation using the *Lactobacillus pentosus* according to (1).
(4) A composition including a biologically pure culture of the *Lactobacillus pentosus* according to (1).

Fermented food or drink products which have an excellent flavor and less quality variation after refrigeration storage can be produced using the lactic acid bacteria of the present invention. Solid fermented food and drink products can be produced because curd forming ability is high if soybeans are used as materials.

### BRIEF DESCRIPTION OF THE DRAWINGS

FIG. 1 is an image of the present strain. FIG. 1A shows a colony image, and FIG. 1B shows a stained image thereof.
FIG. 2 is a graph of viable bacterial count of a culture of Examples and Comparative Examples. FIG. 2A shows measurement results at the start and finish times of the culture. FIG. 2B shows measurement results during refrigeration.
FIG. 3 is a graph of the pH of the culture of Examples and Comparative Examples. FIG. 3A shows measurement results at the start and finish times of the culture. FIG. 3B shows measurement results during refrigeration.
FIG. 4 is a graph of the acidity expressed in terms of lactic acid of the culture of Examples and the Comparative Examples. FIG. 4A shows measurement results at the start and finish times of the culture. FIG. 4B shows measurement results during refrigeration.

### DETAILED DESCRIPTION OF THE INVENTION

In the following, the present invention will be explained in detail.

### <Acquisition of Lactobacillus pentosus strain FERM BP-10958>

Sibazuke collected in the Ohara area of Sakyo-ku, Kyoto was finely diced into 5 mm cubes or smaller using a sterilized scalpel, and was aseptically diluted with saline progressively through a 10-fold dilution series in eight steps to obtain a series of eight dilution samples.

1 mL of the dilution samples was added dropwise to a petri dish, and an MRS agar medium containing 0.5% (w/v) precipitated calcium carbonate was used to produce an agar plate aseptically by a pour plate method, followed by anaerobic culturing at 30°C for 48 hours.

After the culturing, the stereoscopic morphology of colonies formed on the center of the agar was observed, and a white lentoid colony having a diameter of about 1 mm was picked up, aseptically stabed into a stab medium for storage (the MRS agar medium containing 0.5% (w/v) of precipitated calcium carbonate) for inoculation, and then anaerobically cultured at 30°C for 24 hours.

The bacterial body grown on the stab medium for storage was picked up with a platinum needle and was suspended in 1 mL of a physiological salt solution.

The suspended physiological salt solution and an MRS agar medium containing 0.5% (w/v) of precipitated calcium carbonate were used to produce an agar flat plate aseptically in accordance with a pour plate method, and then anaerobic culturing was performed at 30°C for 48 hours.

After the culturing, the stereoscopic morphology of colonies formed on the center of the agar was observed, and a lentoid colony having a diameter of about 1 mm was taken out, aseptically stabbed into a stab medium for storage (the MRS agar medium containing 0.5% (w/v) of precipitated calcium carbonate) for inoculation, and then anaerobically cultured at 30°C for 24 hours.

The bacterial body grown on the stab medium for storage was aseptically picked up with a platinum needle, streaked onto a medium for streak culturing (BL agar medium), and then anaerobically cultured at 30°C for 48 hours.

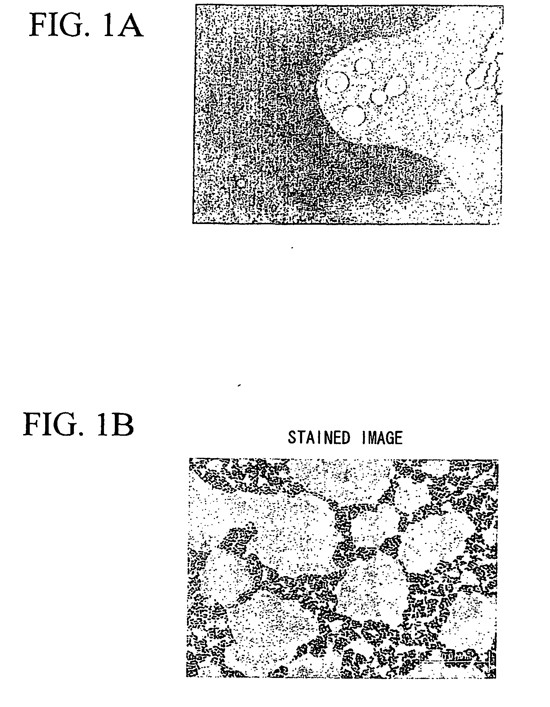

After the culturing, the surface morphology of colonies was examined, and a rounded and smoothly-rimmed colony rising in a semicircular shape and having a color tone from the center to the rim becoming reddish brown or white was selected to obtain *Lactobacillus pentosus* FERM BP-10958 (hereinafter sometimes further abbreviated as "present strain"). A captured image of the present strain at this time is shown in FIG 1. The conditions for capturing images are as follows. Colony image: a close-up picture was taken at a distance of 3 cm form the lens, using a digital camera COOLPIX 4500 (manufactured by Nikon Corporation). Stained image: a picture was taken using a digital camera DP70 (manufactured by Olympus Corporation) and a microscope BX51 (manufactured by Olympus Corporation) and a 100x oil immersion object lens.

### <Identification of Lactobacillus pentosus strain FERM BP-10958>

### (Morphological Property)

The morphological properties of the present strain were checked by microscopic observation of colonies cultured in each media mentioned above. The results revealed that the present strain has a rod-shaped cell morphology, and neither spore nor motility in the same manner as that of *Lactobacillus pentosus* strain JCM1558^{T} (hereinafter sometimes abbreviated as "type strain")

### (Physiological property)

The physiological properties of the present strain were evaluated by a commonly known method and were compared with those of the type strain. The results are shown in Table 1.

As shown in Table 1, the present strain showed the same physiological properties as those of the type strain.

**[Table 1]**

| | | Type strain | Present strain |
|---|---|---|---|
| Gram stainability | | + | + |
| Catalase | | - | - |
| Acid production from glucose | | + | + |
| Gas production from glucose | | + | + |
| Growth | 15°C | + | + |
| | 45°C | - | - |

| | | | |
|---|---|---|---|
| "+" indicates positive and "-" indicates negative | | | |

### (Degradability of Carbohydrates)

The degradability of various types of carbohydrates of the present strain were evaluated using the kit for bacteria identification test, Api 50 CH (manufactured by bio Merieux S.A), and were compared with those of the type strain. The results are shown in Table 2 and Table 3.

As in apparent from Table 2 and Table 3, differences in the assimilabilities of D-xylose and D-raffinose were observed between the present strain and the type strain (the difference is shown by "*" in the Tables).

**[Table 2]**

| No. | Substrate | Type strain | Present strain |
|---|---|---|---|
| 0 | Control | - | - |
| 1 | Glycerol | + | + |
| 2 | Erythritol | - | - |
| 3 | D-arabinose | - | - |
| 4 | L-arabinose | + | + |
| 5 | Ribose | + | + |
| 6 | D-xylose* | + | - |
| 7 | L-xylose | - | - |
| 8 | Adonitol | - | - |
| 9 | β-methyl-D-xylose | - | - |
| 10 | Galactose | + | + |
| 11 | D-glucose | + | + |
| 12 | D-fructose | + | + |
| 13 | D-mannnose | + | + |
| 14 | L-sorbose | - | - |
| 15 | Rhamnose | - | - |
| 16 | Dulcitol | - | - |
| 17 | Inositol | - | - |
| 18 | Mannitol | + | + |
| 19 | Sorbitol | + | + |
| 20 | α-methyl-D-mannoside | - | - |
| 21 | α-methyl-D-glucoside | - | - |
| 22 | N-acethyl-glucosamine | + | + |
| 23 | Amygdalin | + | + |
| 24 | Arbutin | + | + |

| | | | |
|---|---|---|---|
| "+" indicates positive and "-" indicates negative | | | |

**[Table 3]**

| No. | Substrate | Type strain | Present strain |
|---|---|---|---|
| 25 | Esculin | + | + |
| 26 | Salicin | + | + |
| 27 | Cellobiose | + | + |
| 28 | Maltose | + | + |
| 29 | Lactose | + | + |
| 30 | Melibiose | + | + |
| 31 | Sucrose | + | + |
| 32 | Trehalose | + | + |
| 33 | Inulin | - | - |
| 34 | Melezitose | - | - |
| 35 | D-raffinose* | - | + |
| 36 | Starch | - | - |
| 37 | Glycogen | - | - |
| 38 | Xylitol | - | - |
| 39 | Gentiobiose | + | + |
| 40 | D-turanose | - | - |
| 41 | D-lyxose | - | - |
| 42 | D-tagatose | - | - |
| 43 | D-fucose | - | - |
| 44 | L-fucose | - | - |
| 45 | D-arabito | - | - |
| 46 | L-arabitol | - | - |
| 47 | Gluconic acid | - | - |
| 48 | 2-ketogluconic acid | - | - |
| 49 | 5-ketogluconic acid | - | - |

| | | | |
|---|---|---|---|
| "+" indicates positive and "-" indicates negative | | | |

### (Nucleotide Sequence)

The nucleotide sequence (partial sequence) of 16S rDNA of the present strain was determined by a commonly known method. Among the determined nucleotide sequences, the nucleotide sequence from 5'end (position 1) to the position 495 is shown in SEQ ID NO: 1. The determined nucleotide sequence was subjected to the homology search of the DNA Data Bank of Japan (BLAST) administered by the Center for Information Biology and DDBJ, National Institute of Genetics, Research Organization of Information and Systems, Inter-University Research Institute Corporation.

As a result, the nucleotide sequence shown in SEQ ID NO: 1 of the present strain showed 99% or more homology to the type strain, in which one base in the position 22 was unknown while the rest were the same as the type strain.

As shown in the above results, comparing the present strain and the type strain, the nucleotide sequence of 16S rDNA showed 99% or more homology, and the morphological properties and the physiological properties were the same, although the degradabilities of carbohydrates were different. Moreover, the present strain was remarkably superior to the type strain in curd forming ability during the culture. Thus, the present strain is apparently different from conventional strains of lactic acid bacteria that belong to *Lactobacillus pentosus.*

From the above, the present strain is a novel strain of lactic acid bacteria which belongs to *Lactobacillus pentosus.*

### <Lactic Acid Bacteria Strain>

*Lactobacillus pentosus* strain FERM BP-10958 in the present invention was deposited in the International Patent Organism Depositary, National Institute of Advanced Industrial Science and Technology, (Central 6, 1-1 Higashi 1-chome, Tsukuba-shi, Ibaraki-ken, JAPAN (postal code number: 305-8566)) as the accession number of FERM BP-10958 (domestic accession number: FERM P-21248) as of March 9, 2007.

The lactic acid bacteria in the present invention have superior curd forming ability with respect to soybean fermentation. In an existing method, the addition of lactose to the medium was necessary to form curd with soybean fermentation, because soybeans contain so little lactose that soybeans were hard to ferment.

However, the lactic acid bacteria in the present invention can ferment soybeans speedy without the addition of lactose, so the lactic acid bacteria is useful to manufacture like solid yogurts.

The lactic acid bacteria in the present invention are useful especially for the fermentation of vegetative raw materials. Usable examples of the vegetative raw materials may include vegetables, fruits, gains, and beans.

Examples of the vegetables may include tomatos, red bell peppers, carrots, cabbages, Chinese cabbages, lettuces, white radishes, spinaches, kales, onions, egg plants, PETIT VERT (trademark, a cross-breed of kale and Brussels sprouts), shiitake mushrooms, and shimeji mushrooms.

Examples of the fruits may include grapefruits, oranges, apples, grapes, strawberries, pineapples, kiwi fruits, guavas, mangos, acerolas, blueberries, pomegranates, peaches, pears, papayas, melons, watermelons, bananas, and figs.

Examples of the grains may include wheat (malt) and rice, and examples of the beans may include soybeans, and peas.

In the present invention, these vegetative raw materials may be used singularly or in combination of two or more types. The optimum combination may be suitably selected according to the product being the object.

The lactic acid bacteria in the present invention are preferably precultured before being used for the fermentation. The preculture may be carried out by commonly known method. Examples thereof include a method in which a commercially available medium for lactic acid bacteria is dissolved in distilled water at a predetermined concentration, followed by sterilization in an autoclave, and then the lactic acid bacteria in the present invention is inoculated therein and subjected to preculture for a predetermined time. The vegetative raw materials listed above and preferably concentrated juice can be used in place of a commercially available medium for lactic acid bacteria. In precultivation, the lactic acid bacteria can be inoculated in a solid medium, but a liquid medium is preferred.

The fermentation condition of the lactic acid bacteria in the present invention may be suitably selected according to the materials to be used and the object. Preferably, the amount of the inoculation of the lactic acid bacteria in the present invention is 0.1 to 10 % by volume, the fermentation temperature is 20 to 40°C, and the fermentation time is 3 to 72 hours.

### <Fermented Food or Drink, and Methods for Producing the Fermented Food or Drink>

The fermented food or drink of the present invention is produced by fermentation of the lactic acid bacteria of the present invention described above.

The method for producing the fermented food or drink of the present invention has a process of fermentation with the lactic acid bacteria of the present invention described above.

If the materials are fermented with the lactic acid bacteria of the present invention, the fermented product may be directly used as a fermented food or drink, or may be treated or processed as required, to be used as a fermented food or drink. The fermentation condition is the same as described above.

The fermentation materials are not specially limited, although the vegetative raw materials described above are preferable. The optimum combination may be suitably selected according to the desired quality.

Moreover, for the fermentation materials, the suitably processing materials can also be used. For example, the vegetative raw materials may be used in a form of a squeezed liquid, a ground or pulverized form, or in processed form such as a concentrated, diluted, or dried form. Specifically, when soybeans are used, a form of soybean milk or a suspension of small fines can be used. In the present invention, fermentation materials in liquid form are especially suitable for use.

Subsidiary materials may be added to the fermented food and drink products of the present invention, to an extent where the effect of the present invention is not impaired. The subsidiary materials are examples thereof other food materials generally used for foods, pH adjusters, spices, and sugar solutions.

For example, the addition of lactose is not indispensable for soybean fermentation with the lactic acid bacteria of the present invention, but lactose may be added for the purpose of adjusting the flavor of the fermented food and drink products. Lactose, for example, can be added by the addition of nonfat dry milk.

Compared with the fermented food or drink products produced by fermentation using the conventional strains of lactic acid bacteria that belong to *Lactobacillus pentosus,* the fermented food or drink products of the present invention have fewer pickle-like odors, are more fragrant, and are more favorable in terms of sensuality. Moreover, the food or drink products have excellent keeping qualities, since the products have little change of properties such as pH and acidity, and the products maintain excellent flavor in refrigeration storage.

### Examples

In the following, the present invention will be explained in more detail by way of examples. However, the present invention is not limited to these.

### <Fermentation Characteristics of Soybeans Medium>

### (Examples)

Concentrated carrot juice with a Brix 42% was diluted with distilled water so as to adjust its pH to 6.4 and the Brix to 12%. The thus produced carrot medium was inoculated with 1 % (v/v) of a cryopreserved bacterial suspension of the present strain, and was cultured at 30°C for 18 hours to effected activation. Then, the resultant product was subcultured under the same conditions, and was used as a preculture solution.

Next, 50 mL of a medium containing 25% (w/v) commercially available soybean powder, 2% (w/v) glucose, and 4% (w/v) fructose, was inoculated with 1% (w/v) of the above-mentioned preculture solution, and then cultivation was performed at 30°C for 9 hours.

Then, the culture was refrigerated at 5°C or 10°C for 21 days.

For the refrigeration, fermentation characteristics of the medium were evaluated as described below. The viable bacterial count in the culture, pH and acidity of the culture were assayed at the start, finish, and refrigeration time of the culture in the following way, and the curd of the culture was visually observed in the following way. Additionally, sensory evaluation of the culture flavor was performed by a total of 20 panelists consisting of 10 men and 10 women.

### (Comparative Examples)

Cultures and evaluations were performed similarly to examples described above except that the type strain was used instead of the present strain.

### (Measuring Procedure)

(1) Viable bacterial count in the culture
   1 ml of the serial dilution culture was dispensed to petri dishes. Pour plates were made by adding about 20 ml of MRS agar medium (manufactured by OXOID Limited) to the petri dishes. The plates were incubated at 30 °C for 2 days, and a viable bacterial count was worked out by counting formed colonies.
(2) pH of the culture

The pH was determined with a pH meter F-52 (manufactured by

### HORIBA Limited).

### (3) Acidity of the culture

Acidity of the culture was determined by a titration method. That is, about 5 g of sample was weighed, and about 500 µl of phenolphthalein was added to the sample. 0.1 N sodium hydrate was added dropwise to the sample, and the addition was ended when the solution was slightly red in color. Acidity was calculated with the titer using the following equation.

Formula for computation: Acidity (%) = (normality of sodium hydrate solution) × (titer of sodium hydrate solution (L)) × (1 g equivalent weight of lactic acid) ÷ (sample weight) × 100

### (4) Curd of the culture

A container was inverted before inoculation and after culture, if curd did not crumbled down after more than 60 seconds, "○" is indicated, if curd crumbled down before 60 seconds, "Δ" is indicated, and if curd was not formed, "×" is indicated in a table.

### (Results of the Evaluation)

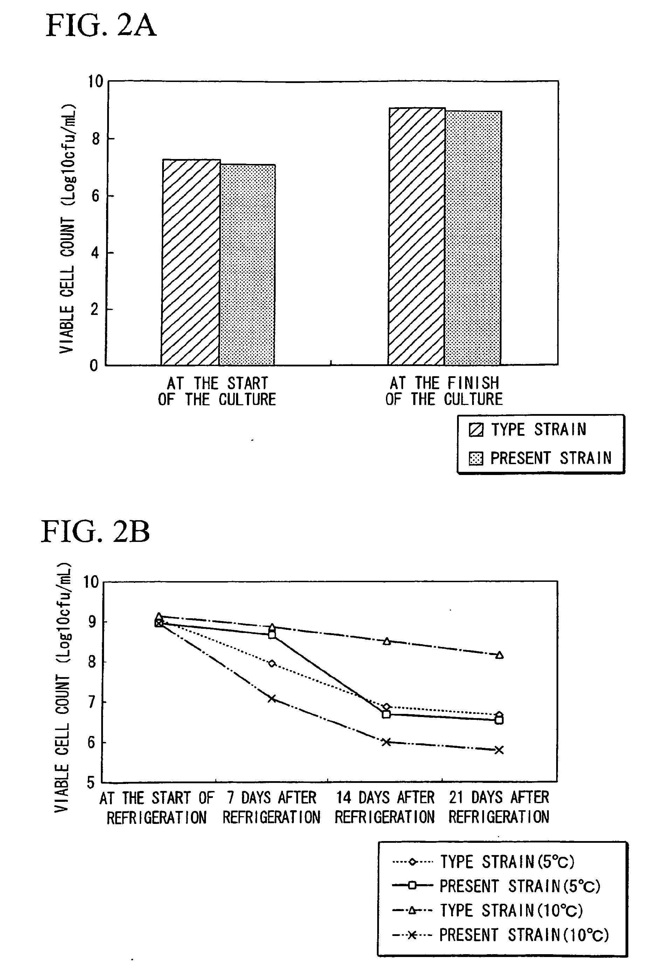

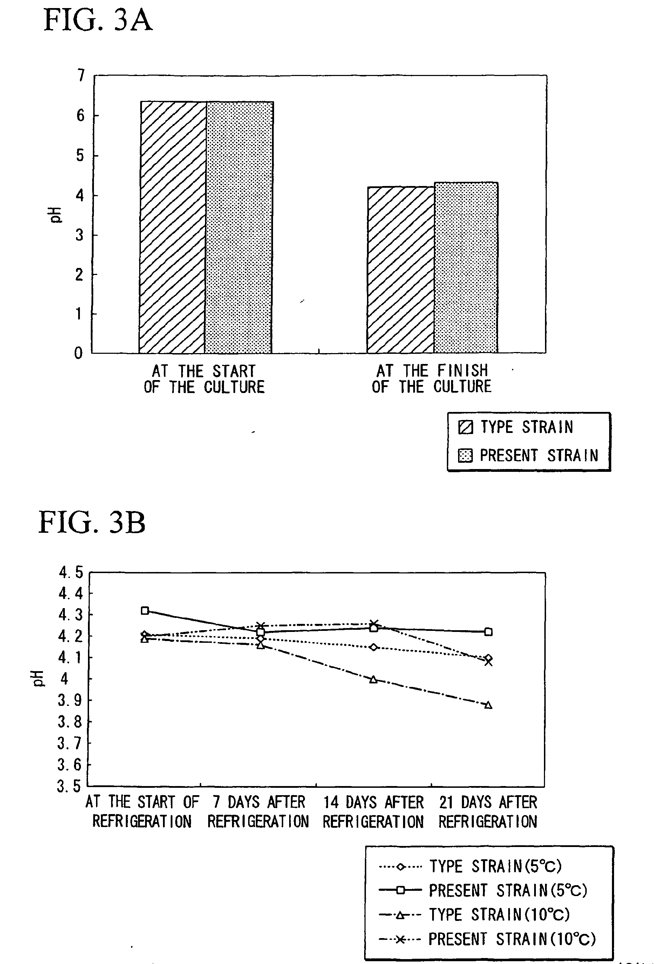

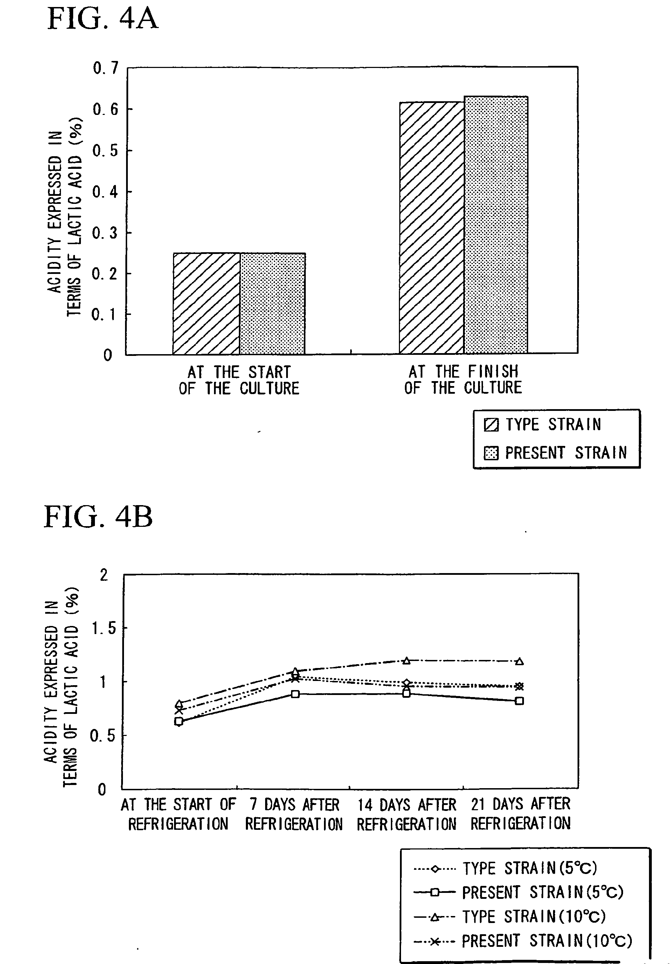

Results of the evaluation are shown in the figures. FIG. 2 is a graph showing a viable bacterial count in the culture. FIG. 3 is a graph showing the pH of the culture. FIG. 4 is a graph of the acidity of the culture. FIG. 2A-FIG. 4A show measurement results at the start and finish times of the culture, and FIG 2B-FIG. 4B show measurement results for the refrigerated period.

### (A) At the start and finish times of the culture

There are no big differences between the present strain and the type strain with respect to growth rate of the viable bacterial count.

There are also no big differences between the present strain and the type strain with respect to changes of the pH and acidity at the start and finish times of the culture.

In contrast, it was shown that the present strain had better curd forming ability than the type strain. The result was considered to be caused by factors other than effects of the acid produced. The results are shown in Table 4.

**[Table 4]**

| (Curd) | Before inoculation | After culture |
|---|---|---|
| Type strain | × | Δ |
| Present strain | × | ○ |

When a flavor of the culture at an ending point was sensory evaluated (Sensory Evaluation 1), the culture of the type strain gave a pickle-like odor. However, the culture of the present strain had no pickle-like odor, and the result was favorable in terms of sensuality. The results are shown in Table 5.

**[Table 5]**

| (Sensory Evaluation 1) | |
|---|---|
| | Number of people who evaluated the flavor as favorable |
| Type strain | 1 |
| Present strain | 19 |

| | |
|---|---|
| * This shows a significant difference at a level of 0.1% significance. | |

### (B) Refrigeration storage

The viable bacterial count of the type strain's culture did not decrease so much, when the culture was stored at 10°C. However, the viable bacterial count clearly decreased when the culture was stored at 5°C. By contrast, the viable bacterial count of the present strain's culture clearly decreased when the culture was stored at not only 5 °C but 10 °C as well.

At 5 °C and 10 °C of storage temperature, changes of pH and acidity of the present strain's culture was lower than the type strain's.

With cultures of the present strain and the type strain, the flavor of a sample, which was frozen just after culture, stored, and defrosted, and the flavor of a sample, which was stored at 10 °C for 21 days, is sensory evaluated (Sensory Evaluation 2). With the culture of the present strain, the flavor of the sample after refrigeration storage almost never changed, and both the sample at culture ending and the sample after storage were preferable. Compared with this, with the culture of the type strain, the flavor changed considerably after storage, and the flavor was not more preferable. The results are shown in Table 6.

From the above results, it was confirmed that the present strain was more temperature-sensitive than the type strain, and there was almost no quality change when the culture was stored at a low temperature.

**[Table 6]**

| (Sensory Evaluation 2) | |
|---|---|
| | Number of people who evaluated the flavor just after culture as favorable |
| Type strain | 17(*) |
| Present strain | 13 |

| | |
|---|---|
| * This shows a significant difference at a level of 0.1% significance. | |

As described above, it was confirmed that the lactic acid bacteria of the present invention had excellent curd forming ability if soybeans were used for the materials, and fermented food and drink products, which had excellent qualities during refrigeration storage and desirable flavor, could be produced by the lactic acid bacteria of the present invention.

The present invention can be used for producing fermented food and drink products.

## Claims

1. *Lactobacillus pentosus* strain FERM BP-10958.

2. A method for producing a fermented food or drink product comprising the step of:
fermenting raw materials of a food or drink product using the *Lactobacillus pentosus* according to Claim 1, in order to obtain a fermented food or drink product.

3. A fermented food or drink product obtained by fermentation using the Lactobacillus *pentosus* according to Claim 1, wherein said food or drink product comprises the
*Lactobacillus pentosus* according to Claim 1.

4. A composition comprising:
a biologically pure culture of the *Lactobacillus pentosus* according to Claim 1.

## Patentansprüche

1. Lactobacillus pentosus-Stamm FERM BP-10958.

2. Verfahren zur Herstellung eines fermentierten Nahrungsmittels oder Getränks, welches den Schritt der Fermentierung von Rohmaterialien eines Nahrungsmittels oder Getränks unter Verwendung von Lactobacillus pentosus gemäß Anspruch 1 umfasst, um ein fermentiertes Nahrungsmittel oder Getränk zu erhalten.

3. Fermentiertes Nahrungsmittel oder Getränk, erhalten durch Fermentierung unter Verwendung von Lactobacillus pentosus gemäß Anspruch 1, wobei das Nahrungsmittel oder Getränk Lactobacillus pentosus gemäß Anspruch 1 umfasst.

4. Zusammensetzung, umfassend eine biologische Reinkultur von Lactobacillus pentosus gemäß Anspruch 1.

## Revendications

1. Souche de *Lactobacillus pentosus* FERM BP-10958.

2. Procédé de production d'un produit de type aliment ou boisson fermenté comprenant l'étape de :
fermentation de matières premières d'un produit de type aliment ou boisson en utilisant le *Lactobacillus pentosus* selon la revendication 1, afin d'obtenir un produit de type aliment ou boisson fermenté.

3. Produit de type aliment ou boisson fermenté obtenu par fermentation en utilisant le *Lactobacillus pentosus* selon la revendication 1, dans lequel ledit produit de type aliment ou boisson comprend le *Lactobacillus pentosus* selon la revendication 1.

4. Composition comprenant :
une culture biologiquement pure de *Lactobacillus pentosus* selon la revendication 1.
